**Europäisches Patentamt**

**European Patent Office**

**Office européen des brevets**

(19)

(11) Veröffentlichungsnummer: **0 293 775**
**A2**

(12) # EUROPÄISCHE PATENTANMELDUNG

(21) Anmeldenummer: **88108481.8**

(22) Anmeldetag: **27.05.88**

(51) Int. Cl.⁴: **C12Q 1/18 , //(C12Q1/18, C12R1:01)**

(30) Priorität: **05.06.87 DE 3718923**

(43) Veröffentlichungstag der Anmeldung: **07.12.88 Patentblatt 88/49**

(84) Benannte Vertragsstaaten: **AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Bansemir, Klaus, Dr.**
**Ursulaweg 51**
**D-4018 Langenfeld(DE)**
Erfinder: **Grebe, Hans-Joachim**
**Moltkestrasse 61**
**D-4000 Düsseldorf 30(DE)**

(54) **Verfahren zum Nachweis der Desinfektionswirkung eines Desinfektionsmittels und hierfür geeigneter Teststreifen.**

(57) Ein Verfahren zum Nachweis der Desinfektionswirkung eines Desinfektionsmittels, bei dem das Desinfektionsmittel auf mit Lumineszenz- oder Leuchtbakterien besiedeltes Filterpapier mit einem Zahlenmittel der Porengröße von 6 bis 8 μ aufgebracht und die Abnahme des Lumineszenz bzw. Leuchtkraft mit derjenigen von unbehandelten Bereichen des Filterpapiers verglichen wird, gestattet eine rasche und unkomplizierte, qualitative oder quantitative Bestimmung der Wirksamkeit der Desinfektion.

Fig. 1

EP 0 293 775 A2

# Verfahren zum Nachweis der Desinfektionswirkung eines Desinfektionsmittels und hierfür geeigneter Teststreifen.

Die Erfindung betrifft ein Verfahren zum Nachweis der Desinfektionswirkung eines Desinfektionsmittels bzw. einer das Desinfektionsmittel enthaltenden Lösung.

Bisher erfolgte der Nachweis der Wirksamkeit eines Desinfektionsmittels durch aufwendige Prüfungen in mikrobiologischen Laboratorien. Entweder durch die Abnahme eines Abdruckes von desinfizierten Flächen mit Rodac-Platten, gefolgt von einer Züchtung der beim Abdruck gewonnenen Bakterien, dies erfordert die Auswertung durch Mikrobiologen, da Sporenbildner erkannt werden müssen (die Sporen können die Desinfektion überstehen), oder nach Testmethoden, wie sie von der DGHM (Deutschen Gesellschaft für Hygiene und Miklrobiologie) verschiedentlich beschrieben wurden. Alle diese Verfahren sind aufwendig; zudem erfordert die Züchtung der Bakterien mindestens eine Zeitspanne von 24 bis 48 Stunden.

Es besteht daher ein Bedürfnis an einem Verfahren zum Nachweis einer Desinfektionsmittelwirkung, das in einfacher Weise und in kürzester Zeit auch von ungeübten Personen durchgeführt werden kann und zuverlässige qualitative oder auch quantita tive Ergebnisse liefert.

Demgemäß ist die Erfindung auf ein Verfahren der eingangs genannten Art gerichtet, bei dem man das Desinfektionsmittel auf mit Lumineszenz- oder Leuchtbakterien besiedeltes Filterpapier mit einem Zahlenmittel der Porengröße von 6 bis 8μ aufbringt und die Abnahme der Lumineszenz bzw. der Leuchtkraft mit derjenigen von unbehandelten Bereichen des Filterpapiers vergleicht.

Damit das so ermittelte Ergebnis eine hinreichend große Aussagekraft hat, ist es erforderlich, daß der Vergleich von mit dem Desinfektionsmittel behandelten und unbehandelten Bereichen auf demselben Filterpapier erfolgt. Weiterhin muß das Filterpapier möglichst gleichmäßig mit den Bakterien besiedelt sein. Überraschenderweise wurde gefunden, daß eine gleichmäßige Besiedelung durch Züchtung dann möglich ist, wenn das Filterpapier das erwähnte Zahlenmittel der Porengröße aufweist. Andere Porengrößenverteilungen ergeben ungleichmäßige Besiedelungsdichten.

Gemäß einer ersten vorteilhaften Ausführungsform der Erfindung beträgt das Zahlenmittel der Porengröße des zu besiedelnden Filterpapiers 7,2 bis 7,6 μ.

Gemäß einer weiteren vorteilhaften Ausführungsform der Erfindung verwendet man ein Filterpapier der vorstehend genannten Art, das mit einer Nährlösung für die Leucht- bzw. Lumineszenzbakterien getränkt ist. In Zusammenwirkung mit einem weiter unten erläuterten Aufbewahrungs- bzw. Transportbehälter erreicht man, daß die Leuchtbakterien auch nach der Ablösung von dem für sie geeigneten Nährmedium eine gewisse Zeitspanne überleben; diese Zeitspanne reicht jedoch zur Durchführung des Verfahrens der Erfindung aus.

Vorzugsweise sind die in dem Verfahren der Erfindung einzusetzenden Lumineszenz- oder Leuchtbakterien aus der von den Gattungen Photobacterium und Lucibacterium gebildeten Gruppe ausgewählt; geeignet sind z.B. Photobacterium phosphoreum, splendidum und mandapamensis sowie Lucibacterium harveyi. Besonders bevorzugt sind Leuchtbakterien der Gattung Photobacterium, wie beispielsweise ein bei der Deutschen Sammlung für Mikroorganismen, Göttingen, unter der Bezeichnung DSM 2167 in der öffentlich zugänglichen Sammlung hinterlegter Mikroorganismus. Geeignet sind auch CCM 2348 und NCMB 844. Darüber hinaus kann der Fachmann Leuchtbakterien erhalten, indem er geeignete Nährböden (z.B. tote Fische) von ubiquitär vorhandenen Leuchtbakterien besiedeln läßt. Dies geschieht, indem diese Nährböden in der freien Umgebung gelagert werden.

Die Erfindung betrifft weiterhin einen Teststreifen zur Verwendung in dem vorgenannten Verfahren, wobei der Teststeifen ein Filterpapier mit einem Zahlenmittel der Porengröße von 6 bis 8 μ, insbesondere 7,2 bis 7,6 μ darstellt, das mit einer Nährlösung für die Leucht- bzw. Lumineszenzbakterien getränkt und mindestens einseitig mit den Leucht- bzw. Lumineszenzbakterien besiedelt ist.

Ein Teststreifen für das Verfahren der Erfindung kann allgemein hergestellt werden, indem man die geeigneten Bakterien züchtet, das Filterpapier in eine Suspension der Bakterien einbringt und nach einiger Zeit entnimmt sowie das präparierte Filterpapier auf eine geeignete Agarplatte auflegt. Anschließend kann das Filterpapier abgenommen und einige Zeit bebrütet werden. Es kann auch in einem verschließbaren Gefäß eine gewisse Zeitspanne aufbewahrt werden, wenn das Gefäß ebenfalls mit einer Agarplatte ausgestattet ist und das mit der Nährlösung getränkte und mit den Bakterien besie delte Filterpapier mit seiner mit den Bakterien besetzten Seite auf der Agarplatte abgesetzt wird.

Zur Duchführung des Verfahrens der Erfindung bringt man einen Tropfen der zu testenden Desinfektionsmittellösung auf das Filterpapier auf. In einem abgedunkelten Raum läßt sich bereits nach einigen Minuten/Sekunden anhand der Abnahme der Leuchtkraft an der behandelten Stelle die Wirksamkeit des Desinfektionsmittels in qualitativer Hinsicht nachprüfen. Weiterhin ist auf diesem Wege eine sichere Unterscheidung zwischen Desinfektionsmittel- und Reinigungsmittellösungen möglich. Man kann den behandelten Teststreifen auch in ein geeignetes optisches Meßinstrument überführen und erhält durch einen Vergleich der Leuchtkraft der behandelten und der unbehandelten Stellen ein quantitatives Ergebnis für die Desinfektionswirkung. Eine Zeitabhängigkeit kann ebenfalls positiv erfaßt werden.

Die Zeichnungen veranschaulichen das Verfahren der Erfindung, insbesondere den hierfür vorgesehenen Teststreifen und dessen Aufbewahrung in einem geeigneten Gefäß. Es zeigen:

Fig. 1 einen Querschnitt durch einem Teststreifen zur Verwendung in dem Verfahren der Erfindung und

Fig. 2 einen Querschnitt durch ein Aufbewahrungs- bzw. Transportgefäß zur Aufnahme des Teststreifens.

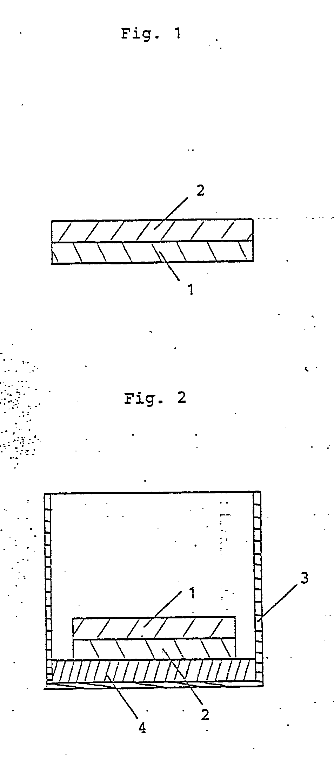

Gemäß Fig. 1 umfaßt der Teststreifen ein Filterpapier 1 mit einem Zahlenmittel der Porengröße von 6 bis 8 μ und eine durch Züchtung aufgebrachte Schicht 2 von Bakterien der Gattung Photobacterium. Der Transport- bzw. Aufbewahrungsbehälter gemäß Fig. 2 umfaßt ein verschließbares Gefäß 3 aus Glas, Kunststoff oder dergleichen mit einem nicht gezeigten Deckel, das an seinem Boden mit einer geeigneten Agarplatte 4 ausgestattet ist. Für die Aufbewahrung bzw. den Transport des Teststreifens 1, 2 wird der mit einer geeigneten Nährlösung getränkte Teststreifen mit der Bakterienbeschichtung 2 nach unten in das Gefäß eingesetzt.

Im folgenden wird die Herstellung der Teststreifen der Erfindung anhand eines bevorzugten Ausführungsbeispiels näher erläutert.

Es wurde zunächst eine Impfsuspension von Bakterien der Gruppe Photobacterium phosphoreum hergestellt. Hierzu wurden die Bakterien bei 16°C auf Krebs-Agar gezüchtet, und zwar unter täglicher Überimpfung mit fraktioniertem Ausstrich unter Auswahl besonders leuchtender Kolonien. Nach 3- bis 5-maliger Passage erfolgte ein Rasenausstrich auf Krebs-Agar, gefolgt von einer 24-stündigen Bebrütung bei 16°C.

Die gezüchteten Kolonien wurden mit jeweils 10 ml Nährlösung nach Krebs pro Platte abgeschwemmt und in einen Weithals-Erlenmeyerkolben mit Glasperlen überführt. Der Kolben wurde zur Sauerstoffsättigung mindestens 1 Stunde mit 150 U/min geschüttelt. Anschließend wurde die Keimsuspension in eine flache Schale überführt. Das Filterpapier wurde 2 Minuten in die Suspension eingetaucht. Anschließend wurde das Filterpapier entnommen und nach Abstreifen der Hauptmenge der Feuchtigkeit luftblasenfrei auf Krebs-Agar aufgelegt. Die Agarplatten wurden zuvor mindestens 1 bis 3 Stunden bei 37°C vorgetrocknet. Das Filterpapier wurde mit einem Glasspatel angedrückt und vorzugsweise 30 bis 60 Minuten bei Umgebungstemperatur stehen gelassen. Anschließend wurde 20 bis 28 Stunden bei 16°C bebrütet.

## Ansprüche

Verfahren zum Nachweis der Desinfektionswirkung eines Desinfektionsmittels bzw. einer das Desinfektionsmittel enthaltenden Lösung, dadurch gekennzeichnet, daß man das Desinfektionsmittel auf mit Lumineszenz- oder Leuchtbakterien besiedeltes Filterpapier mit einem Zahlenmittel der Porengröße von 6 bis 8 μ aufbringt und die Abnahme der Lumineszenz bzw. der Leuchtkraft mit derjenigen von unbehandelten Bereichen des Filterpapiers vergleicht.

2. Verfahren nach Anspruch 1, dadurch gekennzeichnet, daß man ein Filterpapier verwendet, daß ein Zahlenmittel der Porengröße von 7,2 bis 7,6 μ aufweist.

3. Verfahren nach Anspruch 1 oder 2, dadurch gekennzeichnet, daß man ein Filterpapier verwendet, das mit einer Nährlösung für die Lumineszenz- bzw. Leuchtbakterien getränkt ist.

4. Verfahren nach einem der Ansprüche 1 bis 3, dadurch gekennzeichnet, daß die Lumineszenz- oder Leuchtbakterien aus der von Photobacterium und Lucibacterium gebildeten Gruppe ausgewählt sind.

5. Verfahren nach einem der Ansprüche 1 bis 4, dadurch gekennzeichnet, daß die Leuchtbakterien aus der Gruppe der bei der Deutschen Sammlung für Mikroorganismen, Göttingen, unter den Nummern S 88, S 2167, CCM 2348 und NCMB 844 hinterlegten Bakterien der Gattung Photobacterium phosphoreum ausgewählt sind.

6. Teststreifen zur Verwendung in einem der Verfahren gemäß einem der Ansprüche 1 bis 5, gekennzeichnet durch ein Filterpapier mit einem Zahlenmittel der Porengröße von 6 bis 8, insbesondere 7,2 bis 7,6 μ, das mit einer Nährlösung für die Leucht- bzw. Lumineszenzbakterien getränkt und mindestens einseitig mit den Bakterien besiedelt ist.

D 7637 EP

Fig. 1

2

1

Fig. 2

1

3

4

2